# EUROPEAN PATENT SPECIFICATION

(11) **EP 0 665 031 B1**
(45) Date of publication and mention of the grant of the patent: **25.08.1999**
(21) Application number: 95850006.8
(22) Date of filing: 10.01.1995
(51) Int. Cl.: A61N 1/05

(54) **Auxiliary body for guiding a stylet into an electrode cable's stylet channel**
Vorrichtung zur Einführung eines Stiletts in einen Stilettkanal eines Elektrodenkabels
Un corps auxilaire pour guider un stylet dans un canal de stylet d'un câble d'électrode

(30) Priority: 27.01.1994 SE 9400254
(43) Date of publication of application: 02.08.1995
(73) Proprietor: Pacesetter AB, 171 95 Solna (SE)
(72) Inventor: Ingram, Roy, Hitchin, Hirts SG4 8PT (GB); Nyman, Per, S-182 64 Djursholm (SE)
(74) Representative: Winblad, Hans Peter

(56) References cited:
- EP-A- 0 574 904
- US-A- 4 243 050
- US-A- 4 732 163
- US-A- 4 800 890
- US-A- 5 186 179

## Description

This invention relates to an auxiliary body for guiding a stylet into an electrode cable's stylet channel in an electrode device, whose proximal end is provided with a connector pin, one end of the auxiliary body being provided with a cylindrical channel for receiving the connector pin, the other end of the auxiliary body being provided with a cavity whose orifice is larger than the cylindrical channel's opening, the cavity being connected to the cylindrical channel and continuously narrowing to a diameter for receiving the stylet.

It is well known that the physician, in implanting an intracardiac or intravascular electrode device in a patient, employs an auxiliary body of the above-described kind in order to facilitate insertion of the relatively long, thin stylet into the electrode cable's stylet channel from the proximal end of the electrode cable cf. PERMATHANE® endocardial lead, model 866F, manufactured by Pacesetter, INC, Sylmar, California, USA. At this point in the procedure, the physician has already made an opening in a vein for the cable and even prepared implantation of the pacemaker. As a result of these preparations, the physician's rubber gloves are bloody, at least in part. So when the stylet is inserted, the stylet could easily become soiled with blood from the gloves. This means that blood could get into the stylet channel, soil the conductor helix and dry there with a glue-like effect, possibly making it necessary to jerk the stylet to remove it from the stylet channel. This could, in turn, cause the part of the electrode cable installed in a vein or the heart to be jerked out of the desired position. To eliminate this problem, the physician, or an assistant, can dry off the stylet with a dry or wet cloth, thereby introducing an additional, possibly disruptive step in the implantation procedure. Wiping off the stylet with a cloth, possibly soaked in sterile water, removes any blood and simultaneously gives the stylet a relatively slippery surface which could make insertion of the stylet into the stylet channel easier. However, this lubricating effect is of brief duration.

The object of the invention is to achieve an auxiliary body of the type described in the introduction above with which the drying off and/or lubrication of a stylet can be performed in a safe and simple manner, without any additional, disruptive step during implantation.

This problem is solved when the auxiliary body's cavity and/or cylindrical channel is/are provided with a stylet drying and/or lubricating means. This structure ensures that the auxiliary body is always free from blood before it is inserted into the stylet channel and also prevents the stylet from getting stuck in the stylet channel. In addition, lubrication of the stylet in this simple manner make it easier for the physician to introduce the stylet into the stylet channel.

According to one refinement of the invention, it is proposed that the drying and/or lubricating means consist of a felt pad. In this way, the means is concentrated to a single unit arranged in the auxiliary body.

As regards an advantageous refinement of the invention, it is proposed that the drying and/or lubricating means be enveloped in a protective membrane, perforatable by the stylet, to preventing the means from drying out.

In one advantageous version of the invention, it is proposed that the drying and/or lubricating means be detachable. This would enable the physician to remove one means and replace it with another.
According to one preferred embodiment of the invention, it is proposed that the felt pad contain alcohol to remove blood from the stylet.

The felt pad can even advantageously contain cottonseed oil which would have a lubricating effect on the stylet. The invention will be described below in greater detail, referring to FIGURES in attached drawings in which:
FIG. 1 shows a longitudinal cross-section through an auxiliary body according to the invention;
FIG. 2 is a side view of the proximal end of an electrode device, according to FIG. 1, with an auxiliary body mounted.

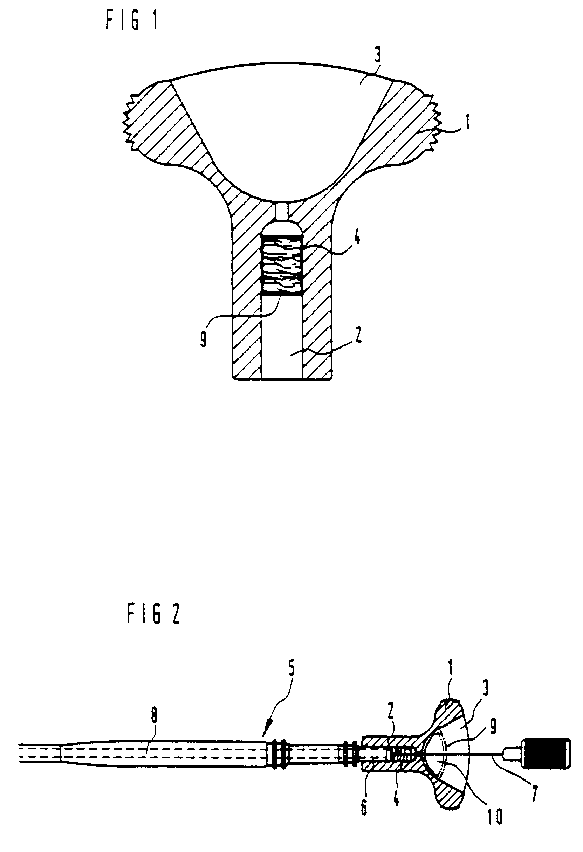

In FIG. 1 is shown an auxiliary body 1 for guiding a stylet into an electrode cable's control channel in an electrode device depicted and described in FIG. 2. One end of the auxiliary body 1 has a cylindrical channel 2, and the other end is provided with a cavity 3, whose opening is larger than the channel 2. The cavity 3 is connected to the channel 2 and continuously narrows in such a way that the diameter of the channel 2 end of the cavity 3 is larger than the diameter of the stylet inserted into the stylet channel and equal to or less than the diameter of the stylet channel. As a result of this structure for the auxiliary body 1, a stylet can be inserted into the said stylet channel in a very simple manner. A felt pad 4 containing a drying means, such as alcohol, or a lubricant, such as cottonseed oil, is arranged in the channel 2. The felt pad 4 is advantageously enveloped in a protective membrane 9, perforatable by the stylet, to prevent the said means from drying out. The felt pad 4 can also be provided with one layer of alcohol and another layer of cottonseed oil, the layers separated by a detachable protective membrane. The felt pad 4 is detachable and therefore replaceable.

In FIG. 2 is shown that the auxiliary body 1, as a result of its cylindrical channel 2, can be pushed onto the said electrode device's 5 connector pin 6. The felt is thereby affixed in the channel 2 by the connector pin 6. The stylet 7 can then be introduced into the stylet channel 8 when the distal end of the stylet is inserted into the cavity 3 in the auxiliary body 1 and the stylet 7 is pushed into the channel 2 through the felt pad 4. When the stylet 7 reaches the stylet channel 8, the stylet 7 has been cleaned and possibly lubricated.

The pad can even be devised to fit inside the cavity 3 in the auxiliary body 1. The dash-dotted lines in the cavity 3 in FIG. 2 indicate the shape and position of such a pad, designated in this example with the reference number 10. One such pad 10 can replace the felt pad 4 arranged in the cylindrical channel 2 in the auxiliary body. The pad, which is enveloped in a protective membrane 9, can advantageously contain a gel for softening and drying off any blood on the stylet 7. With the aid of the gel, the stylet 7 can, when inserted into the cavity 3, be easily straightened in same and then introduced into the stylet channel 8 in the ordinary way.

The pad 10 can also be used in combination with the felt pad 4. In this combination, the pad 10 can suitably contain a softening and drying means in the form of a gel, and the felt pad 4 can contain a lubricating means. Alternately, the pad 10 can contain a softening means, the felt pad 4 then having a drying off function.

With the use of the said felt pad 4 and the felt pad 4 plus the gel pad 10 respectively, which can contain a drying and/or lubricating means, the stylet 7 can be simply and safely cleaned of blood and possibly lubricated before the stylet is inserted into the electrode device's 5 stylet channel 8 during an electrode implantation.

## Claims

1. An auxiliary body for guiding a stylet (7) into an electrode cable's stylet channel (8) in an electrode device (5), whose proximal end is provided with a connector pin, one end of the auxiliary body (1) being provided with a cylindrical channel (2) for receiving the connector pin (6), the other end of the auxiliary body (1) being provided with a cavity whose orifice is larger than the channel's opening, the cavity (3) being connected to the cylindrical channel (2) and continuously narrowing to a diameter for receiving the stylet, **characterized** in that the cavity (3) and/or cylindrical channel (2) is provided with a stylet drying and/or lubricating means (4).

2. An auxiliary body of claim 1, **characterized** in that the drying and/or lubricating means consist of a felt pad (4).

3. An auxiliary body of claim 1 or 2, **characterized** in that the drying and/or lubricating means (4) is enveloped in a protective, perforatable membrane (9).

4. An auxiliary body of any of claims 1-3, **charazterized** in that the drying and/or lubricating means (4) is removable.

5. An auxiliary body of any of claims 1-4, **characterized** in that the felt pad (4) contains an alcohol.

6. An auxiliary body of any of claims 1-4, **characterized** in that the felt pad (4) contains cottonseed oil.

## Patentansprüche

1. Ein Hilfsmittel zum Einführen eines Mandrins (7) in den Mandrinkanal (8) eines Elektrodenkabels in einer Elektrodenvorrichtung (5), deren proximales Ende mit einem Anschlußstift versehen ist, wobei das eine Ende des Hilfsmittels (1) mit einem zylindrischen Kanal (2) zur Aufnahme des Anschlußstiftes (6) und das andere Ende des Hilfsmittels (1) mit einer Ausnehmung versehen ist, deren Öffnung größer als die Öffnung des Kanals ist, wobei die Ausnehmung (3) mit dem zylindrischen Kanal (2) verbunden ist und sich kontinuierlich auf einen Durchmesser zur Aufnahme des Mandrins verjüngt, dadurch gekennzeichnet, daß die Ausnehmung (3) und/oder der zylindrische Kanal (2) mit einem Reinigungs- und/oder Gleitmittel (4) für den Mandrin versehen ist.

2. Ein Hilfsmittel nach Anspruch 1, dadurch gekennzeichnet, daß das Reinigungs- und/oder Gleitmittel aus einem Filzkissen (4) besteht.

3. Ein Hilfsmittel nach Anspruch 1 oder 2, dadurch gekennzeichnet, daß das Reinigungs- und/oder Gleitmittel (4) von einer perforierbaren Schutzschicht (9) umgeben ist.

4. Ein Hilfsmittel nach einem der Ansprüche 1-3, dadurch gekennzeichnet, daß das Reinigungs- und/oder Gleitmittel (4) abnehmbar ist.

5. Ein Hilfsmittel nach einem der Ansprüche 1-4, dadurch gekennzeichnet, daß das Filzkissen (4) Alkohol enthält.

6. Ein Hilfsmittel nach einem der Ansprüche 1-4, dadurch gekennzeichnet, daß das Filzkissen (4) Baumwollsamenöl enthält.

## Revendications

1. Corps auxiliaire pour guider un stylet (7) dans un canal (8) de stylet de câble d'électrode dans un dispositif (5) d'électrode, dont l'extrémité proximale est munie d'une broche de connecteur, une extrémité du corps (1) auxiliaire étant munie d'un canal (2) cylindrique pour recevoir la broche (6) de connecteur, l'autre extrémité du corps (1) auxiliaire étant munie d'une cavité dont l'orifice est plus grand que l'ouverture du canal, la cavité (3) étant reliée au canal (2) cylindrique et devenant de manière continue plus étroite jusqu'à un diamètre destiné à recevoir le stylet, caractérisé en ce que la cavité (3) et/ou le canal (2) cylindrique est/sont muni(s) de moyens (4) de lubrification et/ou de séchage de stylet.

2. Corps auxiliaire suivant la revendication 1, caractérisé en ce que les moyens de séchage et/ou de lubrification sont constitués d'un tampon (4) de feutre.

3. Corps auxiliaire suivant la revendication 1 ou 2, caractérisé en ce que les moyens (4) de lubrification et/ou de séchage sont enveloppés dans une membrane (9) protectrice pouvant être perforée.

4. Corps auxiliaire suivant l'une quelconque des revendications 1 à 3, caractérisé en ce que les moyens (4) de séchage et/ou de lubrification sont amovibles.

5. Corps auxiliaire suivant l'une quelconque des revendications 1 à 4, caractérisé en ce que le tampon (4) de feutre contient un alcool.

6. Corps auxiliaire suivant l'une quelconque des revendications 1 à 4, caractérisé en ce que le tampon (4) de feutre contient de l'huile de coton.
